# EUROPEAN PATENT APPLICATION

(11) **EP 4 269 365 A1**
(43) Date of publication of application: **01.11.2023**
(21) Application number: 21911118.4
(22) Date of filing: 27.12.2021
(51) Int. Cl.: C02F 3/00, C02F 3/12

(54) **METHOD FOR TREATING ORGANIC WASTEWATER**

(30) Priority: 25.12.2020 JP 2020217913
(71) Applicant: Kubota Corporation, Osaka 556-8601 (JP)
(72) Inventor: WAKAHARA, Shinichiro, Amagasaki-shi, Hyogo 661-8567 (JP); SUZUKI, Nobukazu, Amagasaki-shi, Hyogo 661-8567 (JP); KOMATSU, Toshihiro, Amagasaki-shi, Hyogo 661-8567 (JP)
(74) Representative: Plasseraud IP
(86) International application number: PCT/JP2021/048523
(87) International publication number: WO 2022/138981

(57) **Abstract**

Provided is a method for treating wastewater, the method being capable, even during low water temperature periods, of efficiently purifying organic wastewater that experiences large temperature differences in a year. A method for treating organic wastewater uses a device including a predetermined reaction tank containing activated sludge and a culture tank and includes the steps of: culturing, in the culture tank, a microorganism contained in the activated sludge, while maintaining a temperature of a content of the culture tank at a temperature that is at least 5°C lower than a water temperature of the organic wastewater at a start of culturing of the microorganism and not lower than -1°C and not higher than 10°C; and then introducing the microorganism into the reaction tank.

## Description

### Technical Field

The present invention relates to a method for treating organic wastewater.

### Background Art

It is known that, in treatment of wastewater such as domestic wastewater or industrial wastewater with use of a microorganism, when the water temperature of the wastewater drops in winter, the activity of the microorganism also decreases as a result, and a resultant decrease in the wastewater treatment performance of the microorganism deteriorates the quality of treated water.

The decrease in wastewater treatment performance causes, for example, generation of scum, foaming, residual ammonia due to a decrease in nitrification activity, an increase in undecomposed residues, a decrease in membrane filterability due to an increase in metabolites, and the like. This deteriorates the quality of treated water.

Examples of a method for solving the decrease in wastewater treatment performance or the deterioration in quality of treated water include: a method in which a flocculant is added to remove a substance that deteriorates membrane filterability; a method in which a microorganism having a relatively high activity in winter is added in the form of a formulation; and a method in which a nutrition that is insufficient in activated sludge is added.

Further, as a method for treating wastewater at low temperatures, Patent Literature 1 discloses a method for stably treating ammonia nitrogen-containing water even at a water temperature of not higher than 15°C, by using a group of nitrifying bacteria cultured in a highly-concentrated ammonium sulfate solution.

Further, Patent Literature 2 discloses a method that removes, even in a low temperature environment, a nitrogen component contained in wastewater by using bacteria resistant to highly-concentrated ammonia.

Patent Literature 3 discloses a water treatment device in which a tank for culturing a microorganism is isolated from wastewater and which is capable of cost reduction by controlling addition of the microorganism in accordance with the amounts of phosphoric acid, ammonia, dissolved oxygen, and the like contained in the wastewater.

### Citation List

### [Patent Literature]

[Patent Literature 1]
   International Publication No. WO2014/017429
[Patent Literature 2]
   Japanese Patent Application Publication, Tokukai, No. 2016-107219
[Patent Literature 3]
   Specification of US Patent No. 7736509

### Summary of Invention

### Technical Problem

In a case where a sufficient capacity of a reaction tank is secured and the water temperature is stably low, a microorganism having a low-temperature resistance can grow in a naturally-occurring manner, and it is thus unlikely that the wastewater treatment performance decreases. However, the water temperature of wastewater typified by domestic wastewater generally exceeds 20°C in summer and decreases to approximately 10°C in winter (except in tropical and subtropical regions). In such a case where the wastewater experiences a significant change in water temperature, a low-temperature resistance acquired by a microorganism is deactivated when the temperature increases. Alternatively, due to a difference in growth rate between a microorganism having a low-temperature resistance and a microorganism not having a low-temperature resistance, the low-temperature resistance of the microorganism tends to disappear.

In a case where the low-temperature resistance of the microorganism is deteriorated and the water temperature of the wastewater falls below 15°C and continues to decrease, undecomposed residues start to accumulate before the microorganism having a low-temperature resistance sufficiently grows or before the activity of the microorganism is improved by adaptation. That is, the growth of the microorganism having a low-temperature resistance and the increase in activity cannot catch up with the decrease in water temperature.

Thus, in a case where wastewater treatment is carried out with use of microorganisms in a certain amount (MLSS concentration), the proportion of a microorganism having a low-temperature resistance gradually decreases in the sludge. This increases a load per microorganism, so that an overloaded microorganism generates metabolites such as an extracellular polymeric substance (EPS) and a biomass (SMP), and/or undecomposed residues are left. This results in problems such as a decrease in membrane filterability and deterioration of the quality of treated water.

In a case where a measure is taken after the water temperature starts to decrease, deterioration of the quality of treated water proceeds because the measure does not necessarily bring about an effect immediately. Thus, it takes a long time and a large amount of cost to improve the quality of treated water again.

With the above-described methods for solving the degradation of wastewater treatment performance, there is a problem of high cost because it is necessary to add a flocculant, a microorganism formulation, or a nutritional agent in each winter. There is another problem that, since the addition is carried out after the quality of treated water deteriorates, it takes a long time until the quality of treated water improves.

In Patent Literature 1, the treatment is limited to a treatment targeting ammonia nitrogen. Further, Patent Literature 1 indicates that the water temperature during the treatment is not lower than 0°C and not higher than 10°C. Patent Literature 1 thus does not recognize a case in which there is a great difference in water temperature. Thus, Patent Literature 1 cannot solve the above-described problems. Similarly, Patent Literature 2 also assumes sewage having a water temperature of not more than 20°C, and does not assume a case in which there is a great difference in water temperature.

Patent Literature 3 simply indicates that it is not necessary to heat or cool the entire device because the microorganism is cultured separately from the wastewater to be treated and is introduced into the wastewater as appropriate. Patent Literature 3 does not assume a temperature difference in wastewater. Regarding culturing of the microorganism, Patent Literature 3 indicates that the microorganism is cultured in an incubator. However, Patent Literature 3 merely describes that many bacteria grow well at approximately 37°C, and does not mention the relationship between the temperature at which the bacteria is cultured and the water temperature of the wastewater. Further, parameters serving as indices for introduction of the microorganism are phosphoric acid, ammonia, dissolved oxygen, and the like in the wastewater, which are merely requirements for growth of the microorganism. The growth of a useful microorganism is not directly confirmed. Thus, at a time point when these parameters change, the quality of the wastewater may already be contaminated.

It is an object of an aspect of the present invention to provide a method for treating organic wastewater, the method being (i) capable, even during low water temperature periods, of purifying organic wastewater that experiences large temperature differences in a year and (ii) capable of preventively avoid degradation of the quality of treated water.

### Solution to Problem

As a result of diligent study, the inventor of the present invention has found that it is possible to solve the foregoing problems by, with use of a device including (i) a reaction tank for treating organic wastewater with use of activated sludge and (ii) a culture tank provided separately from the reaction tank, specifying a temperature at which a microorganism is cultured in the culture tank and a condition for introducing the microorganism thus cultured into the reaction tank. The inventor has thereby completed the present invention.

That is, the present invention includes the following configurations.
<1>A method for treating organic wastewater with use of a device including (i) a reaction tank for treating organic wastewater with use of activated sludge and (ii) a culture tank provided separately from the reaction tank,
   the method including the steps of:
   (A) culturing, in the culture tank, a microorganism contained in the activated sludge, while maintaining a temperature of a content of the culture tank at a temperature that is (i) at least 5°C lower than a water temperature of the organic wastewater at a start of culturing of the microorganism and (ii) not lower than -1°C and not higher than 10°C; and
   (B) introducing, into the reaction tank, the microorganism which has been subjected to the step (A).
<2>The method as set forth in <1>, wherein the temperature of the content of the culture tank is not higher than a minimum water temperature of the organic wastewater in a year.
<3>The method as set forth in <1> or <2>, wherein a time of culturing of the microorganism is longer than a doubling time of the microorganism.
<4>The method as set forth in any one of <1> through <3>, wherein the step (B) is a step of introducing the microorganism from the culture tank into the reaction tank, (B-1) at a timing that precedes, by at least 1 times a sludge residence time, a day on which a minimum water temperature of the organic wastewater is expected to be below 15°C or (B-2) in a case where an index biomass in the reaction tank becomes equal to a threshold or exceeds the threshold.
<5>The method as set forth in <4>, wherein the index biomass is: a value of soluble chemical oxygen demand (S-COD) concentration; or a ratio of a mixed liquor volatile suspended solid (MLVSS) to a mixed liquor suspended solid (MLSS).
<6>The method as set forth in <5>, wherein: in a case where the index biomass is the value of the S-COD concentration, the threshold is 100 mg/L; and in a case where the index biomass is the ratio of the mixed liquor volatile suspended solid (MLVSS) to the mixed liquor suspended solid (MLSS), the threshold is a value obtained by increasing, by 5%, a reference value measured during one month centering on a day on which the organic wastewater has a maximum water temperature in a year.
<7>The method as set forth in any one of <4> through <6>, wherein the index biomass is monitored by a monitoring system.
<8>The method as set forth in any one of <1> through <7>, wherein a water temperature of the organic wastewater is such that: there is a difference of at least 5°C between a maximum water temperature and a minimum water temperature in a year; and the minimum water temperature in a year is not lower than 4°C and lower than 15°C.
<9>The method as set forth in any one of <1> through <8>, further including the following step (C) which is carried out after the step (B):
   (C) detecting an amount of the microorganism in the reaction tank and, in a case where the amount is less than a threshold, further introducing the microorganism from the culture tank into the reaction tank.
<10>The method as set forth in claim 9, wherein the amount of the microorganism is detected by: an analysis using an amount of 16S rRNA in the microorganism as an index; an analysis using real-time PCR; an antigen-antibody reaction; or a DNA aptamer method.

### Advantageous Effects of Invention

An aspect of the present invention makes it possible to provide a method for treating organic wastewater, the method being (i) capable, even during low water temperature periods, of purifying organic wastewater that experiences large temperature differences in a year and (ii) capable of preventively avoid deterioration of the quality of treated water.

### Brief Description of Drawings

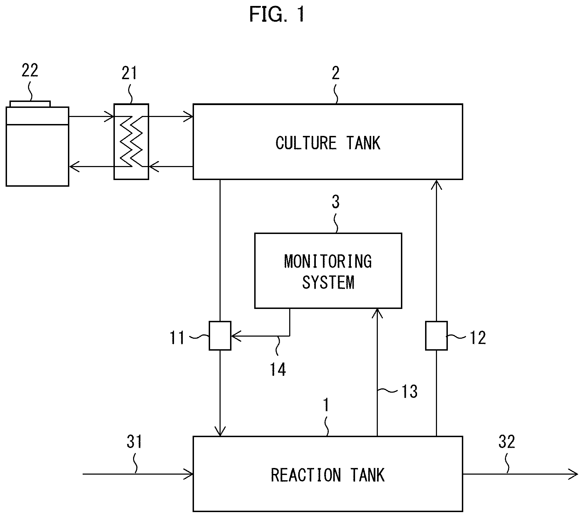

Fig. 1 is a schematic view illustrating an example of a wastewater treatment device which is used in a method for treating organic wastewater in accordance with an embodiment of the present invention.

### Description of Embodiments

The following will describe an embodiment of the present invention in detail. Note, however, that the present invention is not limited to the following embodiment, but can be altered within this disclosure. For example, the present invention also encompasses, in its technical scope, any embodiment derived by combining technical means disclosed in differing embodiments. Note that a numerical range "A to B" herein means "not less than A and not more than B" unless otherwise specified in the present specification.

### [1. Method for treating organic wastewater]

A method for treating organic wastewater in accordance with an embodiment of the present invention (hereinafter also referred to as "present treatment method") is a method for treating organic wastewater with use of a device including (i) a reaction tank for treating organic wastewater with use of activated sludge and (ii) a culture tank provided separately from the reaction tank,
the method including the steps of:
(A) culturing, in the culture tank, a microorganism contained in the activated sludge, while maintaining a temperature of a content of the culture tank at a temperature that is (i) at least 5°C lower than a water temperature of the organic wastewater at a start of culturing of the microorganism and (ii) not lower than -1°C and not higher than 10°C; and
(B) introducing, into the reaction tank, the microorganism which has been subjected to the step (A).

As described above, in a case where a low-temperature resistance of a microorganism is deteriorated, taking a measure after the water temperature of the organic wastewater falls below 15°C cannot prevent deterioration of the quality of treated water from proceeding, because an effect of the measure is not necessarily exhibited immediately.

In contrast, according to the above configuration, the treatment of organic wastewater is started with use of a microorganism that has been cultured in a low temperature environment in advance. As such, even in a case where the water temperature of the organic wastewater falls below 15°C, it is possible to carry out treatment of the organic wastewater while maintaining the wastewater treatment capability of the microorganism at a high level. This makes it possible to efficiently separate the activated sludge from the purified treated water and to maintain the quality of the treated water at a good level.

A method for treating organic wastewater in accordance with an embodiment of the present invention (hereinafter also referred to as "the present treatment method") is carried out with use of a device for treating organic wastewater (hereinafter also referred to as "the present treatment device"). Fig. 1 is a schematic view illustrating an example of the present treatment device. The present treatment device will be described below with reference to Fig. 1. As illustrated in Fig. 1, the present treatment device includes: a reaction tank 1 that treats organic wastewater with use of activated sludge; and a culture tank 2 provided separately from the reaction tank.

The reaction tank 1 is a tank for treating organic wastewater 31 with use of the activated sludge. The reaction tank 1 aerates the organic wastewater introduced into the reaction tank 1 and the activated sludge in the reaction tank 1 to purify the organic wastewater with use of the microorganism contained in the activated sludge. As the reaction tank 1, a reaction tank conventionally used in an activated sludge process can be used.

Although not illustrated in Fig. 1, the purified treated water and the activated sludge are separated by separation using a final sedimentation tank, membrane filtration, or the like, and the treated water is discharged as treated water 32. The reaction tank 1 may be coupled to the culture tank 2 via a circulation pump 12 that collects activated sludge from the inside of the reaction tank 1.

Note here that it is preferable that the separation of the purified treated water from the activated sludge be carried out by membrane filtration. A membrane activated sludge process in which the separation is carried out by membrane filtration has the following advantages: management of activated sludge is easy; activated sludge in the reaction tank can be maintained at a high concentration; the facility can be made compact because there is no need for a final sedimentation tank; and the like.

Meanwhile, the increase in undecomposed residues, the increase in metabolites, and the like described above are caused by a decrease in the wastewater treatment performance of the microorganism, and it is therefore too late to take a measure after the minimum water temperature of the organic wastewater falls below 15°C. This problem is particularly significant in the membrane activated sludge process. It has been difficult to stably treat organic wastewater also in winter while taking advantage of the above-described advantages.

The present treatment method starts treatment of organic wastewater with use of a microorganism that has been cultured in a low-temperature environment in advance. Thus, it is possible to stably treat the organic wastewater even in a case where the minimum water temperature of the organic wastewater falls below 15°C. This makes it possible to efficiently carry out the treatment of organic wastewater while maintaining the advantages of the membrane activated sludge process.

The culture tank 2 is a tank for culturing a microorganism contained in the activated sludge. As the culture tank 2, for example, a horizontal cylindrical tank made of FRP can be used. The temperature of a content of the culture tank 2 is at least 5°C lower than the water temperature of the organic wastewater at the start of culturing of the microorganism and is -1°C to 10°C.

The content of the culture tank 2 is cooled in a heat exchanger 21 with use of cooling water delivered from a chiller 22, in order to maintain the temperature range described above. The culture tank 2 may be coupled to the reaction tank 1 by an introduction pump 11 capable of delivering the content of the culture tank 2.

In a case where the reaction tank 1 is made of reinforced concrete and the culture tank 2 is installed at the same time as the reaction tank 1, the culture tank 2 may be made of reinforced concrete similarly to the reaction tank 1. Note, however, that the present embodiment is not limited to this. Further, in a case where the culture tank 2 is installed after the reaction tank 1 is installed, a horizontal cylindrical water tank made of FRP, a vertical cylindrical water tank made of PE, or the like may be used as the culture tank 2. Note, however, that the present embodiment is not limited to this.

The device for treating organic wastewater may further include a monitoring system 3. The monitoring system 3 is capable of controlling ("14" in Fig. 1) the amount of the microorganism introduced by the introduction pump 11 into the reaction tank 1, for example, by making an analysis of an index biomass in the activated sludge collected ("13" in Fig. 1) from the reaction tank 1 and predicting a state of the organic wastewater after the time point of making the analysis. This point will be described later. Note that the collection of sludge can be carried out manually or automatically.

The following will describe steps of the present treatment method in detail.

The type of organic wastewater is not particularly limited, and can be industrial wastewater, domestic wastewater (for example, sewage), landfill leachate, or the like, or wastewater that is a mixture of any ones of these types of wastewater.

The treatment process to which the present treatment method is applied is not particularly limited, and can be, for example, a batch activated sludge process or a continuous activated sludge process. The continuous activated sludge process may be, for example, any of the following: a standard activated sludge process; an extended aeration process; an oxidation ditch process; a two-stage aeration process; an anaerobic-aerobic process; a circulating anaerobic-aerobic process; a nitrification liquid circulation activated sludge process (denitrification activated sludge process; a nitrification denitrification process); and a membrane activated sludge process. In particular, as described above, the membrane activated sludge process is preferable.

### (1-1. Step (A))

The step (A) in the present treatment method cultures, in the culture tank, a microorganism contained in the activated sludge, while maintaining a temperature of a content of the culture tank at a temperature that is (i) at least 5°C lower than a water temperature of the organic wastewater at a start of culturing of the microorganism and (ii) not lower than -1°C and not higher than 10°C.

The activated sludge contains a microorganism for use in treatment of organic wastewater. As used herein, "microorganism" means at least one kind of organism which can ordinarily be included in activated sludge. The microorganism is preferably selected from the group consisting of bacteria, archaeobacteria, and fungi.

The microorganism can be a species that inherently has a low-temperature resistance, or can be a microorganism that has acquired a low-temperature resistance by going through a cold shock by being cultured at a low temperature (e.g., not higher than 10°C) or temporarily cultured in a low temperature environment. The bacteria preferably belongs to the genus Bacillus, and examples of such bacteria include *Bacillus subtilis.*

As used herein, "having a low-temperature resistance" means being capable of growing in an environment with a temperature of not higher than 10°C, preferably not higher than 5°C.

The use of the microorganism contained in the activated sludge makes it possible to carry out culturing of the microorganism without newly introducing a microorganism formulation or the like. Further, since the activated sludge in the reaction tank is circulated, the microorganism having low-temperature resistance easily becomes a dominant species.

As the activated sludge, for example, activated sludge collected from the reaction tank can be used. A method of collecting the activated sludge from the reaction tank and introducing the activated sludge into the culture tank is not particularly limited. For example, the activated sludge can be collected from the reaction tank with use of a circulation pump connecting the reaction tank and the culture tank, and then the activated sludge can be introduced into the culture tank.

The activated sludge may inevitably include organic wastewater. That is, for example, it can be only a solid component of the activated sludge that is introduced into the culture tank, or a mixture of organic wastewater and the solid component can be introduced into the culture tank.

In the step (A), the temperature of the content of the culture tank is (i) at least 5°C lower than the water temperature of the organic wastewater at the start of culturing of the microorganism and (ii) not lower than -1°C and not higher than 10°C.

According to the above-described configuration, the microorganism is cultured in advance at a low temperature. Thus, the microorganism is given a so-called cold shock. As such, even in a case where (i) the microorganism is added to the organic wastewater before the water temperature of the organic wastewater falls below 15°C and (ii) then the water temperature falls below 15°C, the activity of the microorganism is less likely to decrease.

The temperature in the culture tank is preferably at least 6°C lower, more preferably at least 7°C lower, than the water temperature of the organic wastewater. The upper limit of the difference between the temperature in the culture tank and the water temperature of the organic wastewater is not particularly limited but may be, for example, not higher than 10°C.

The temperature of the content of the culture tank is preferably not lower than -1°C and not higher than 8°C, and more preferably not lower than -1°C and not higher than 5°C. In a case where the temperature of the content of the culture tank is not higher than 10°C, the microorganism easily has low-temperature resistance.

The water temperature of the organic wastewater at the start of culturing of the microorganism can be obtained by actual measurement. Further, for example, by setting the heat exchanger 21 and the chiller 22 so that the temperature of the content of the culture tank is (i) at least 5°C lower than the water temperature and (ii) not lower than -1°C and not higher than 10°C, it is possible to cause the temperature of the content of the culture tank to be (i) at least 5°C lower than the water temperature and (ii) not lower than -1°C and not higher than 10°C.

The temperature of the content of the culture tank in the step (A) is preferably not higher than a minimum water temperature of the organic wastewater in a year.

For example, in a case where the water temperature of the organic wastewater at the start of culturing of the microorganism is 13°C, a value that is 5°C lower than the water temperature is 8°C. In this case, by setting the temperature of the content of the culture tank to be 8°C, it is possible to satisfy the following requirement described above: "(i) at least 5°C lower than the water temperature of the organic wastewater at the start of culturing of the microorganism and (ii) not lower than -1°C and not higher than 10°C".

Meanwhile, in a case where the minimum water temperature of the organic wastewater in a year is 7°C, it is preferable that the temperature of the content of the culture tank be 7°C rather than 8°C. This is because, causing the microorganism to adapt to an expected minimum water temperature of the organic wastewater, into which the microorganism is to be introduced, makes it possible for the microorganism to decompose an organic substance more efficiently in the organic wastewater.

The "minimum water temperature of the organic wastewater in a year" means a minimum water temperature, in a year, of organic wastewater flowing into the reaction tank in which the organic wastewater is treated. In the present specification, "a year" means a period from April 1 to March 31 of the following year.

The "minimum water temperature" is preferably, but not necessarily limited to, a minimum water temperature of organic wastewater that has a composition identical to or similar to that of the organic wastewater to be treated. For example, a minimum water temperature of tap water, industrial water, or the like may be regarded as a minimum water temperature of the organic wastewater.

The "minimum water temperature in a year" may be a minimum water temperature obtained on the basis of data of daily changes in water temperature in only the past year. The "minimum water temperature in a year" may also be an average value of minimum water temperatures obtained on the basis of data of daily changes in water temperature in a past plurality of years.

The data of daily changes in water temperature preferably pertains to as many years as possible. Further, the data of daily changes in water temperature preferably pertains to years that are close to a year to which the day of carrying out treatment of organic wastewater belongs. For example, it is preferable that the minimum water temperature in a year be an average value of minimum water temperatures determined from respective sets of data, obtained over the past 5 years, of daily changes in water temperature in a year.

Further, the person who measures the data is not particularly limited. For example, the data may be data measured by the same person who carries out the treatment of the organic wastewater. Further, the minimum water temperature in a year may be, for example, a value estimated from air temperature data measured by an organization such as a local meteorological observatory.

As described above, even in a case where the water temperature is low, the activity of the microorganism is unlikely to decrease, provided that the water temperature is stable throughout the year. In a case where the water temperature of the organic wastewater decreases rapidly, on the other hand, the activity and/or amount of the microorganism in the organic wastewater decreases rapidly.

Note, here, that in a case where a microorganism that is in the activated sludge and has not been subjected to the step (A) is subjected to the step (B) (described later), the microorganism cannot exhibit its function quickly. Further, in the step (B-1) (described later), the microorganism is introduced into the organic wastewater before the day on which the minimum water temperature of the organic wastewater falls below 15°C. At this time, in a case where the microorganism is introduced in a state where the microorganism does not have low-temperature resistance, the viability rate of the microorganism decreases.

In the step (A), the microorganism in the culture tank has been cultured in advance at a low temperature and thus has a low-temperature resistance. As such, the activity and/or amount of the microorganism are/is not reduced even in a case where the microorganism is introduced into the organic wastewater having a reduced water temperature. It is thus possible to prevent a decrease in organic wastewater treatment capability at a low water temperature in winter.

The time of culturing of the microorganism in the step (A) is preferably not less than a doubling time of the microorganism. Though depending on a type of microorganism, a type of culture medium, and the like, the time of culturing of the microorganism in the step (A) is preferably at least two times, more preferably as least five times, even more preferably at least 10 times the doubling time.

In a case where the culturing time is not less than the doubling time of the microorganism, the amount of a microorganism having low-temperature resistance is sufficiently increased. This improves the organic wastewater treatment capability in a case where the microorganism is introduced into the reaction tank. The upper limit of the culturing time is not particularly limited.

The "doubling time of the microorganism" means a mean generation time and can be determined by finding a growth curve obtained by plotting (i) a microorganism count obtained from a turbidity or the like and (ii) a time.

The timing at which the step (A) is started is not particularly limited but may be determined on the basis of a timing at which the step (B) (described later) is started. That is, for example, the step (A) may be started so that the doubling time ends by the timing at which the step (B) (described later) is started. In a case where the timing at which the step (A) is started is determined on the basis of the timing at which the step (B) is started, it is possible to efficiently carry out each step.

In the step (A), the dissolved oxygen (DO) in the culture tank had a concentration of preferably not less than 2 mg/L, more preferably not less than 3 mg/L, and even more preferably not less than 5 mg/L. In a case where the DO concentration is not less than 2 mg/L, it is possible to further improve the activity of the microorganism. The upper limit of the DO is not particularly limited but may be, for example, not more than 10 mg/L.

### (1-2. Step (B))

The present treatment method includes the step (B) of introducing, into the reaction tank, the microorganism which has been subjected to the step (A). As stated above, the microorganism has acquired low-temperature resistance through the step (A). Thus, the microorganism can quickly exhibit its function after being introduced into the reaction tank.

The step (B), for example, introduces the microorganism from the culture tank into the reaction tank, (B-1) at a timing that precedes, by at least 1 times a sludge residence time, a day on which a minimum water temperature of the organic wastewater is expected to be below 15°C or (B-2) in a case where an index biomass in the reaction tank becomes equal to a threshold or exceeds the threshold.

As described above, it is too late to take a measure after the quality of the treated water has deteriorated. In such a case, it takes a long time and a large amount of cost to improve the quality of the treated water. In contrast, by carrying out the step (B-1) or the step (B-2) to prevent the activity and/or amount of the microorganism from being reduced by a decrease in water temperature, it is possible to preventively avoid deterioration of the quality of treated water. This makes it possible to stably carry out treatment of organic wastewater even in winter.

In a case where the step (B-1) is carried out in the step (B), the microorganism is introduced at a timing that precedes, by at least 1 times a sludge residence time, a day on which a minimum water temperature of the organic wastewater is expected to be below 15°C. With this configuration, the microorganism can be introduced before a low temperature period in which the activity of the microorganism decreases. That is, the step (B-1) is characterized by starting the introduction of the microorganism in advance of a timing at which the organic wastewater has a low water temperature.

Note that, in a case where the organic wastewater has a low organic substance concentration, the sludge residence time may be as long as, for example, 400 days. In this case, it is not economical to introduce the microorganism at a timing that precedes, by at least 1 times a sludge residence time, a day on which the minimum water temperature of the organic wastewater is expected to be below 15°C, because the introduction of the microorganism is far in advance of the day on which the minimum water temperature is expected to be below 15°C. As such, it is preferable that whether or not to carry out the step (B-1) be appropriately determined in consideration of the organic substance concentration of the organic wastewater and the like. In the case of this example, it is considered preferable to adopt, for example, the step (B-2).

Examples of a method for determining a day on which the minimum water temperature of the organic wastewater is expected to be below 15°C (hereinafter also referred to as "low water temperature day") include a method in which a water temperature change in the organic wastewater is predicted on the basis of data of daily changes in the past and the low water temperature day is determined.

Examples of the data of daily changes in the past include data of daily changes, in a year, in water temperature of organic wastewater flowing into the reaction tank in which treatment of organic wastewater is carried out. The data may be data of daily changes in only a year or may be data of daily changes in water temperature in a plurality of years.

Further, the low water temperature day may be determined on the basis of, for example, the above-described air temperature data or the like or may be determined on the basis of a prediction of the monitoring system (described later).

As described above, the step (B-1) is carried out before the low water temperature day. As such, the water temperature of the organic wastewater at the time of introduction of the microorganism is not lower than 15°C, preferably not lower than 16°C, and more preferably not lower than 17°C.

As used herein, a "sludge residence time (SRT)" means a time it takes for all of the activated sludge in the reaction tank to be replaced. An SRT can be determined by dividing the amount of the activated sludge in the reaction tank by the amount of surplus activated sludge that is drawn out per day.

Though depending on a raw water concentration, a water temperature, a "hydraulic residence time (HRT)", or the like, a preferable SRT is typically 2 days to 40 days in the activated sludge process in which sludge is concentrated in a final sedimentation tank. In the membrane activated sludge process (MBR process), the SRT is 5 days to 200 days, and preferably 20 days to 100 days.

As used herein, a "hydraulic residence time (HRT)" means a time it takes for organic wastewater, activated sludge, or the like to flow out of the reaction tank after flowing into the reaction tank. The HRT can be determined by dividing the volume of the reaction tank by the amount of activated sludge or the like that flows into the reaction tank per hour.

In the step (B-1), the microorganism is introduced at a timing that precedes the low water temperature day by at least 1 times the SRT. The timing of introducing the microorganism precedes the low water temperature day by preferably at least 1.5 times the SRT, more preferably at least 2 times the SRT, and even more preferably at least 2.5 times the SRT.

By introducing the microorganism at a timing that precedes the low water temperature day by at least 1 times the SRT, it is possible to secure a sufficient amount of the microorganism by the low water temperature day and to efficiently treat the organic wastewater in accordance with a decrease in water temperature.

Thus, it is possible to prevent accumulation of undecomposed residues that occurs when the water temperature decreases. Further, it is possible to prevent membrane fouling caused by metabolites such as EPS or SMP. Note that, in terms of cost, the microorganism may be introduced later than the timing that precedes the low water temperature day by at least three times the SRT.

In order to save the labor of calculating the SRT, the microorganism may be introduced at a timing that is between one week before the low water temperature day and one month before the low water temperature day. As a value of SRT, it is preferable to use a value on the low water temperature day. In a case where there are a plurality of low water temperature days in a year, it is preferable to use an average value of respective SRTs of the low water temperature days. Note here that "a year" means a period from April 1 to March 31 of the following year, as described above.

As the SRT, an average value of a plurality of SRTs in a week centering on a low water temperature day may be used. In a case where there are a plurality of low water temperature days in a year, the SRT may be an average value of SRTs during a period from the first low water temperature day in the year (from April 1 to March 31 of the following year) to the last low water temperature day in the year. Alternatively, the SRT may be an average value of SRTs during a period from three days before the first low water temperature day in that one year to three days after the last low water temperature day in the year. The SRT may be an average value of SRTs during one week centered on a day that is in the middle of a period from the first low water temperature day in the year to the last low water temperature day in the year.

The value of the SRT may be a value, such as a median value, that is representative of SRTs in a period during which measurement of SRT is carried out.

In a case where the step (B-2) is carried out in the step (B), the microorganism cultured in the step (A) is introduced from the culture tank into the reaction tank in a case where the index biomass in the reaction tank becomes equal to a threshold or exceeds the threshold.

In the step (B-2), the introduction of the microorganism is carried out with reference to an index biomass at a certain point in time, not with reference to the quality of the organic wastewater. Thus, the microorganism is introduced before the quality of treated water actually deteriorates or the membrane filterability actually decreases. Thus, it is possible to preventively avoid the quality of treated water from deteriorating.

That is, it is preferable that the microorganism be immediately introduced in a case where the index biomass becomes equal to the threshold or exceeds the threshold. Further, in the step (B-2), it is possible to introduce the microorganism only in a required amount, since the introduction of the microorganism is carried out on the basis of the index biomass. Thus, it is possible to reduce the amounts of the microorganism, the culture medium, and the like which are used.

As used herein, the "index biomass" is a value of an index representing the state of organic wastewater, activated sludge, or the like in the reaction tank. Examples of the value include: a value of a mixed liquor suspended solid (MLSS) which is an index of the amount of activated sludge in the reaction tank; a value of a mixed liquor volatile suspended solid (MLVSS) which indicates the amount of an organic substance in the reaction tank, and a value of a chemical oxygen demand (COD) which is an index of the degree of pollution caused by an organic substance.

In the step (B-2), it is preferable that the index biomass be: a value of soluble chemical oxygen demand (S-COD) concentration; or a ratio of a mixed liquor volatile suspended solid (MLVSS) to a MLSS. These parameters facilitate obtaining accurate numerical values in the resident water, so that the step (B-2) can be carried out at a more appropriate timing.

In a case where the value of the S-COD concentration is used as an index biomass, the threshold is preferably 100 mg/L, more preferably 50 mg/L, and even more preferably 30 mg/L. In this case, it is preferable that the microorganism be introduced from the culture tank into the reaction tank in a case where the value of the S-COD concentration becomes equal to the threshold or exceeds the threshold. Note that the term "exceeds the threshold" will be described later.

The S-COD concentration can be measured, for example, by centrifuging organic wastewater in the reaction tank to obtain a supernatant and subjecting the supernatant to S-COD concentration measurement with use of a COD meter, a colorimetric method, or the like.

Further, for example, organic wastewater collected from the reaction tank may be subjected to suction filtration with use of the five classes C filtration paper for quantitative analysis described in JIS P 3801-1995 to obtain a filtration liquid, and a COD concentration of the filtration liquid may be used as an index biomass.

In a case where the ratio of MLVSS to MLSS is used as an index biomass, it is preferable that the threshold be a relative value rather than an absolute value. Since MLVSS varies depending on the region where the raw water is located, using a relative value makes it possible to calculate an index biomass more accurately.

In a case where the threshold is a relative value, a reference value of the ratio is preferably measured during a month centering on a day on which the maximum water temperature in a year is expected to be recorded (hereinafter referred to as "a maximum water temperature day in a year").

The "maximum water temperature day in a year" is a day on which the above-described "maximum water temperature of organic wastewater in a year" is predicted to be recorded.

The term "a month centering on a maximum water temperature day in a year" can mean a period from 15 days before a maximum water temperature day in a year to 15 days after the maximum water temperature day in the year, or can mean a period from 14 days before the maximum water temperature day in a year to 14 days after the maximum water temperature day in the year.

In a case where there are a plurality of maximum water temperature days in a year, the term "a month centering on a maximum water temperature day in a year" can mean a period from 15 days or 14 days before the first maximum water temperature day in the year to 15 days or 14 days after the last maximum water temperature day in the year. Further, the term "a month centering on a maximum water temperature day in a year" can mean a month centering on a day in the middle of a period from the first maximum water temperature day in the year to the last maximum water temperature day in the year.

A reference value of the ratio may be an average value of ratios in the above-described period. The reference value of the ratio may be a value, such as a median value, that is representative of ratios in a period during which measurement is carried out. The maximum water temperature day in a year may be a day determined on the basis of data on the maximum water temperature day in only the past year. Alternatively, the maximum water temperature day in a year may be a day determined by calculation on the basis of data on maximum water temperature days in a plurality of years in the past.

By measuring the ratio during a period determined in this way and calculating an average value and the like of ratios thus measured, it is possible to determine a reference value of the ratio. The ratio is preferably measured on each day in the period at a time of the day when a maximum water temperature is expected to be recorded.

The threshold is a value which is increased, preferably by 5%, more preferably by 3%, even more preferably by 2%, from a reference value of the ratio. In this case, it is preferable that the microorganism be introduced from the culture tank into the reaction tank in a case where the value of the S-COD concentration becomes equal to the threshold or exceeds the threshold.

For example, an average value of ratios of MLVSS to MLSS measured in a period from 15 days before the maximum water temperature day in a year to 15 days after the maximum water temperature day in the year is used as a reference value. Then, it is preferable that the microorganism be introduced into the reaction tank when a ratio of MLVSS to MLSS at a certain time point is a value increased from the average value by not less than 5%.

In a case where the threshold of the index biomass is the above value, it is possible to carry out the step (B) at an appropriate timing. This is because in a case where the threshold is exceeded, it is predicted that the quality of treated water will deteriorate soon.

The MLSS is a value measured as a suspended solid by subjecting the organic wastewater in the reaction tank to a method stipulated in "14.1 Suspended solid" in JIS K 0102:2019. The MLVSS is a value measured as a loss on ignition by subjecting the suspended substance to a method stipulated in "14.5 Loss on ignition" of JIS K 0102:2019. A ratio of MLVSS to MLSS can be determined by dividing the obtained value of MLVSS by the value of MLSS.

The value of the index biomass can vary from site to site where treatment of organic wastewater is carried out. As such, it is preferable that the threshold of the index biomass be set individually for each reaction tank in which treatment of organic wastewater is carried out.

As used herein, "exceeding a threshold" means that a value of a parameter defined as an index biomass is either above or below a certain numerical value defined as a threshold. Whether "exceeding a threshold" means being "above" or "below" the certain numerical value depends on the index biomass.

For example, suppose the threshold is determined to be "x". In a case where it is not desirable, in relation to deterioration of water quality, that the value of the parameter be above the numerical value x, it is determined that the threshold is exceeded when the value of the parameter is above the numerical value x. Conversely, in a case where it is not desirable, in relation to deterioration of water quality, that the value of the parameter be below the numerical value x, it is determined that the threshold is exceeded when the value of the parameter is below the numerical value x.

For example, in a case where the value of S-COD is used as a threshold and in a case where the ratio of MLVSS to MLSS is used as a threshold, it is determined that the threshold is exceeded when the value of the parameter is above the threshold.

In the present treatment method, the index biomass in the reaction tank may be monitored by the monitoring system. Continuous monitoring by the monitoring system enables immediate introduction of the microorganism in a case where the index biomass becomes equal to a threshold or exceeds the threshold.

The monitoring system is not particularly limited and can be a known device used for wastewater treatment. Further, the monitoring system can monitor the index biomass with respect to any of activated sludge in the reaction tank, organic wastewater, and a mixture thereof. Further, the index biomass monitored by the monitoring system may be a value of the S-COD, a ratio of MLVSS to MLSS, or the like.

The introduction of the microorganism from the culture tank into the reaction tank can be carried out with use of a conventionally known introduction pump or the like. Further, the amount of the microorganism introduced from the culture tank into the reaction tank may be determined as appropriate on the basis of, for example, the degree of contamination of the organic wastewater.

The monitoring system may control the amount of the microorganism introduced. The amount of the microorganism introduced, can be controlled by, for example, a method in which the amount of the microorganism in the culture tank introduced is controlled on the basis of the index biomass by interference with an introduction pump that connects the culture tank and the reaction tank.

The organic wastewater treated in the present treatment method has a difference of not less than 5°C between a maximum water temperature and a minimum water temperature in a year, and has a minimum water temperature in a year of not less than 4°C and lower than 15°C.

Organic wastewater that satisfies this condition undergoes a significant change in water temperature. As such, in a case where the water temperature falls below 15°C and continues to decrease, undecomposed residues start to accumulate before the microorganism having a low-temperature resistance sufficiently grows or before the activity of the microorganism is improved by adaptation. Thus, it is impossible to prevent deterioration of the water quality if a measure is taken after the water temperature has fallen below 15°C. That is, the organic wastewater is difficult to treat by an ordinary method.

The organic wastewater has a difference between a maximum water temperature and a minimum water temperature in a year of preferably not less than 5°C, more preferably not less than 7°C, and even more preferably not less than 10°C.

Further, the minimum water temperature of the organic wastewater in a year is preferably not less than 4°C and not more than 13°C, more preferably not less than 4°C and not more than 10°C, and even more preferably not less than 4°C and not more than 8°C. The maximum water temperature of the organic wastewater is not particularly limited but may be, for example, not lower than 20°C and not higher than 40°C.

Note that the "minimum water temperature of organic wastewater in a year" has already been described.

The "maximum water temperature of organic wastewater in a year" is explained by replacing the term "minimum water temperature" in "minimum water temperature in a year of organic wastewater" described above with a maximum water temperature.

Thus, that "a water temperature of the organic wastewater is such that: there is a difference of at least 5°C between a maximum water temperature and a minimum water temperature in a year; and the minimum water temperature in a year is not lower than 4°C and lower than 15°C" means that there is a difference of at least 5°C between a maximum water temperature of organic wastewater flowing into the reaction tank in which treatment of the organic wastewater is carried out and a minimum water temperature of the organic wastewater; and the minimum water temperature in a year is not lower than 4°C and lower than 15°C.

That is, organic wastewater to be treated itself does not have measured values of the maximum water temperature in a year and the minimum water temperature in a year. As such, the maximum water temperature in the year and the minimum water temperature in the year of the organic wastewater to be treated are not actual measured values but predicted values which can be obtained, for example, from the above-described data.

### (1-3. Step (C))

The present treatment method can further include the following step (C) which is carried out after the step (B): (C) detecting an amount of the microorganism in the reaction tank and, in a case where the amount is less than a threshold, further introducing the microorganism from the culture tank into the reaction tank.

Since the present treatment method includes the step (C), it is possible to prevent the quality of treated water from being deteriorated by, for example, a decrease in amount of the microorganism after the completion of the step (B) due to, for example, imbalance of nutrients in the wastewater.

The step (C) may be carried out only once or may be carried out a plurality of times. The step (C) is preferably carried out a plurality of times. It is particularly preferable that the step (C) be carried out every time the amount of the microorganism in the reaction tank is below the threshold. In a case where the step (C) is carried out a plurality of times, it is possible to stably maintain a good quality of treated water.

In the step (C), it is preferable that the amount of the microorganism be detected by: an analysis using an amount of 16S rRNA in the microorganism as an index; an analysis using real-time PCR; an antigen-antibody reaction; or a DNA aptamer method. Note that the threshold of the amount of the microorganism can be set as follows. That is, the threshold can be set by: initializing the threshold on the basis of a value of the amount of a microorganism in another similar treatment plant; accumulating measured data on the quality of organic wastewater to be treated and the relationship between filterability and the amount of the microorganism; and updating the threshold to a value sufficient to satisfy organic wastewater treatment performance.

With the use of these methods, it is easy to accurately identify the amount of the microorganism and thus to appropriately carry out the step (C). Further, these methods may be carried out by collecting wastewater in the reaction tank or may be carried out automatically with use of the monitoring system. These methods are preferably carried out with use of the monitoring system because, as described above, it is possible to immediately carry out the step (C).

### Examples

The present invention will be described in more detail with reference to examples below. Note, however, the present invention is not limited to the following Examples.

### [Example 1]

Treatment of organic wastewater was carried out with use of a microorganism which had been cultured at low temperature. The wastewater treatment process used was an MBR process of a circulating nitrification-denitrification type. Sewage (of a separate sewage system) was used as the organic wastewater. SRT was 6 hours. The sewage had a water temperature of 20°C.

Activated sludge collected from the sewage was put in a culture tank and cultured for 24 hours while the temperature of the activated sludge was maintained at 10°C. During the culturing, DO in the culture tank was always maintained at more than 2 mg/L. As the culture tank, a shaking incubator and a 250-ml baffled triangular flask were used.

After the culturing was completed, the activated sludge containing the microorganism was introduced into the sewage. At the time of introduction of the activated sludge containing the microorganism, 50 mL of an LB culture medium diluted 20-fold was added to the sewage as a substrate. Subsequently, a soluble total organic carbon (S-TOC) concentration was measured at the time of the introduction and 24 hours after the introduction, respectively, with use of TOC-L (manufactured by Shimadzu Corporation). Then, on the basis of the S-TOC values thus measured, a diminution rate of S-TOC per hour was measured.

### [Comparative Example 1]

A diminution rate of S-TOC in the sewage was measured in the same manner as in Example 1 except that the temperature of the activated sludge was set to 20°C.

The results of Example 1 and Comparative Example 1 are shown in Table 1. In the table, "culture temperature" is a temperature of activated sludge in the culture tank. "Water temperature of wastewater" is a water temperature of the sewage. "Elapsed time 0 h S-TOC (mg/L)" is S-TOC at the time of introduction of the activated sludge. "Elapsed time 24 h S-TOC (mg/L)" is S-TOC as of 24 hours after the introduction of the activated sludge. "Diminution rate (mgTOC/L/h)" is a rate of diminution in S-TOC per hour.

**[Table 1]**

| | Example 1 | Comparative Example 1 |
|---|---|---|
| Culture temperature (°C) | 10 | 20 |
| Water temperature of wastewater (°C) | 20 | 20 |
| Elapsed time 0 h S-TOC (mg/ L) | 587 | 580 |
| Elapsed time 24 h S-TOC (mg/ L) | 448 | 452 |
| Diminution rate (mgTOC/ L/h) | 5.79 | 5.33 |

From Table 1, it is understood that, in the wastewater into which the microorganism was introduced in Example 1, the diminution rate of TOC is approximately 1.1 times that of Comparative Example 1.

### [Example 2]

A diminution rate of S-TOC in the sewage was measured in the same manner as in Example 1 except that the water temperature of the sewage was set to 11°C and the temperature of the activated sludge was set to 6°C.

### [Comparative Example 2]

A diminution rate of S-TOC in the sewage was measured in the same manner as in Example 2 except that the temperature of the activated sludge was set to 11°C.

The results of Example 2 and Comparative Example 2 are shown in Table 2. The meanings of the words in the table are the same as in Table 1.

**[Table 2]**

| | Example 2 | Comparative Example 2 |
|---|---|---|
| Culture temperature (°C) | 6 | 11 |
| Water temperature of wastewater (°C) | 11 | 11 |
| Elapsed time 0 h S-TOC (mg/ L) | 616 | 604 |
| Elapsed time 24 h S-TOC (mg/ L) | 447 | 462.6 |
| Diminution rate (mgTOC/ L/h) | 7.04 | 5.89 |

From Table 2, it is understood that the diminution rate of TOC in Example 2 is approximately 1.2 times that of Comparative Example 2.

The results shown in Tables 1 and 2 above have shown that applying a cold shock to the microorganism improves the diminution rate of TOC, i.e., the speed of treatment of the wastewater. It was also shown that the lower the water temperature of the wastewater and the culture temperature of the microorganism, the greater the effect of the cold shock. As such, it can be said that culturing the microorganism in advance at a low temperature as in the present treatment method is effective for improving the activity of the microorganism, in particular, at a low temperature.

The present treatment method includes the step (A) of culturing a microorganism at a temperature that is (i) at least 5°C lower than the water temperature of the organic wastewater at the start of culturing of the microorganism and (ii) not lower than -1°C and not higher than 10°C. In view of the results of the above-described Examples, it can be considered that the use of the present treatment method makes it possible to improve the activity of a microorganism in a low water temperature period, and thus can contribute to inhibition of accumulation of undecomposed residues, inhibition of metabolites, and the like. Thus, it is expected that membrane filterability does not easily deteriorate, and deterioration of the quality of treated water can therefore be prevented even during low water temperature periods.

### Industrial Applicability

The present invention can be utilized in treatment of sewage such as organic wastewater, a sewage treatment plant, a purification tank, a landfill leachate treatment, an industrial wastewater treatment, and the like.

### Reference Signs List

1: Reaction tank
2: Culture tank
3: Monitoring system
11: Introduction pump
12: Circulation pump
13: Collection of sludge
14: Flow rate control
21: Heat exchanger
22: Chiller
31: Organic Wastewater
32: Treated water

## Claims

1. A method for treating organic wastewater with use of a device including (i) a reaction tank for treating organic wastewater with use of activated sludge and (ii) a culture tank provided separately from the reaction tank,
the method comprising the steps of:
(A) culturing, in the culture tank, a microorganism contained in the activated sludge, while maintaining a temperature of a content of the culture tank at a temperature that is (i) at least 5°C lower than a water temperature of the organic wastewater at a start of culturing of the microorganism and (ii) not lower than -1°C and not higher than 10°C; and
(B) introducing, into the reaction tank, the microorganism which has been subjected to the step (A).

2. The method as set forth in claim 1, wherein the temperature of the content of the culture tank is not higher than a minimum water temperature of the organic wastewater in a year.

3. The method as set forth in claim 1 or 2, wherein a time of culturing of the microorganism is longer than a doubling time of the microorganism.

4. The method as set forth in any one of claims 1 through 3, wherein the step (B) is a step of introducing the microorganism from the culture tank into the reaction tank, (B-1) at a timing that precedes, by at least 1 times a sludge residence time, a day on which a minimum water temperature of the organic wastewater is expected to be below 15°C or (B-2) in a case where an index biomass in the reaction tank becomes equal to a threshold or exceeds the threshold.

5. The method as set forth in claim 4, wherein the index biomass is: a value of soluble chemical oxygen demand (S-COD) concentration; or a ratio of a mixed liquor volatile suspended solid (MLVSS) to a mixed liquor suspended solid (MLSS).

6. The method as set forth in claim 5, wherein:
in a case where the index biomass is the value of the S-COD concentration, the threshold is 100 mg/L; and
in a case where the index biomass is the ratio of the mixed liquor volatile suspended solid (MLVSS) to the mixed liquor suspended solid (MLSS), the threshold is a value obtained by increasing, by 5%, a reference value measured during one month centering on a day on which the organic wastewater has a maximum water temperature in a year.

7. The method as set forth in any one of claims 4 through 6, wherein the index biomass is monitored by a monitoring system.

8. The method as set forth in any one of claims 1 through 7, wherein a water temperature of the organic wastewater is such that: there is a difference of at least 5°C between a maximum water temperature and a minimum water temperature in a year; and the minimum water temperature in a year is not lower than 4°C and lower than 15°C.

9. The method as set forth in any one of claims 1 through 8, further comprising the following step (C) which is carried out after the step (B):
(C) detecting an amount of the microorganism in the reaction tank and, in a case where the amount is less than a threshold, further introducing the microorganism from the culture tank into the reaction tank.

10. The method as set forth in claim 9, wherein the amount of the microorganism is detected by: an analysis using an amount of 16S rRNA in the microorganism as an index; an analysis using real-time PCR; an antigen-antibody reaction; or a DNA aptamer method.
